# EUROPÄISCHE PATENTSCHRIFT

(11) **EP 0 444 305 B1**
(45) Veröffentlichungstag und Bekanntmachung des Hinweises auf die Patenterteilung: **21.09.1994**
(21) Anmeldenummer: 90125333.6
(22) Anmeldetag: 22.12.1990
(51) Int. Cl.: A61B 10/00

(54) **Vorrichtung zur Probeentnahme mittels Biopsie**
Sampling device by biopsy
Dispositif pour le prélèvement d'un échantillon par biopsie

(30) Priorität: 28.02.1990 DE 4006175
(43) Veröffentlichungstag der Anmeldung: 04.09.1991
(73) Patentinhaber: ANGIOMED AG, 76227 Karlsruhe (DE)
(72) Erfinder: Schnepp-Pesch, Wolfram, W-7505 Ettlingen (DE); Lindenberg, Josef, W-7500 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 238 461
- WO-A-83/03343
- DE-U- 8 802 580
- US-A- 4 766 907

## Beschreibung

Die Erfindung betrifft eine Vorrichtung zur Probeentnahme mittels Biopsie, mit einer Hohlnadel und einem in dieser geführten Stilett, die beide federbeaufschlagt sind, wobei die Hohlnadel unter Federkraft nach vorne schiebbar ist.

Eine gattungsgemäße Vorrichtung ist aus der EP-A-238 461 bekannt. Diele Vorrichtung weist als Stilett eine sogenannte "Tru-cut-Nadel" auf, die eine seitliche Aufnahme aufweist. Die Tru-cut-Nadel wird unter Wirkung einer ersten Feder in den Biopsiebereich eingeschossen, wodurch sich Gewebe in die seitliche Ausnehmung hineindrückt. Anschließend wird die Hohlnadel in der gleichen Richtung über die Ausnehmung geschossen, wodurch das in die Ausnehmung eingedrückte Gewebe abgeschnitten wird und durch Tru-cut-Nadel und Hohlnadel gehalten wird und derart gemeinsam durch beide aus dem Körper dem Kern und der weiteren Untersuchung zugeführt werden kann. Eine ähnlich funktionierende, aber komplizierter aufgebaute Vorrichtung ist aus der WO83/03343 bekannt. Die EP-A-10 321 sowie die EP-A-153 047 zeigen Vorrichtungen, bei denen das die seitliche Einkerbung aufweisende Stilett von Hand vorgeschoben und lediglich die Hohlnadel unter Federwirkung über diese geschossen wird.

Der Erfindung liegt die Aufgabe der Schaffung einer verbesserten Vorrichtung zur Probeentnahme mittels Biopsie zugrunde.

Erfindungsgemäß wird die genannte Aufgabe mit einer Vorrichtung zur Probeentnahme mittels Biopsie, mit einer Hohlnadel und einem in dieser geführten Stilett, die beide federbeaufschlagt sind, wobei die Hohlnadel unter Federkraft nach vorne schiebbar ist, gelöst, welche dadurch gekennzeichnet ist, daß zur Erzeugung eines Unterdrucks innerhalb der Hohlnadel das Stilett vor dem Vorschießen der Hohlnadel unter Federkraft zurückziehbar ist.

Die erfindungsgemäße Vorrichtung geht damit einen anderen Weg als die Vorrichtung nach dem Stand der Technik. Es werden nicht nacheinander zunächst das Stilett und dann die Hohlnadel über das Stilett geschossen. Die Erfindung zieht vielmehr das Stilett unter Federwirkung vor dem Vorschießen der Hohlnadel mit der Stilettspitze in die Hohlnadel zurück, anschließend wird die Hohlnadel in an sich bekannter Weise unter Federkraft vorgeschossen, schneidet aber dabei im Unterschied zum Stande der Technik einen vollzylindrischen Pfropfen aus dem Gewebe aus, welcher dann durch den durch das Zurückziehen des Stiletts in der Hohlnadel bewirkten Unterdruck in dieser in die Hohlnadel hineingezogen und von dem umgebenden Gewebe gelöst wird. Durch diese erfindungsgemäße Probeentnahme kann ein gleiches Gewebevolumen bzw. ein Gewebeteil mit gleichem Gewebequerschnitt, wie beim Stand der Technik, mit einem Stilett und einer Hohlnadel mit wesentlich geringeren Querschnittsabmessungen gewonnen werden, als dies beim Stand der Technik der Fall ist. Damit ist die Belastung für den derart zu untersuchenden Patienten wesentlich geringer, was insbesondere bei empfindlichen Körperteilen oder Organen wesentlich ist. Wenn auch grundsätzlich die Vorrichtung eine Tru-cut-Nadel aufweisen könnte, deren seitliche Ausnehmung bei der erfindungsgemäßen Vorrichtung nicht genutzt wird, so ist in bevorzugter Ausgestaltung vorgesehen, daß das Stilett eine glatte durchgehende zylindrische Wandung ohne jegliche seitliche Aufnehmung aufweist. Lediglich seine Spitze wird angespitzt sein, beispielsweise in Form eines Trokarschliffes. Die Hohlnadel wird an ihrer Stirnseite ebenfalls geschärft sein, wobei die Stirnseite der Hohlnadel vorzugsweise nicht insgesamt abgeschrägt zu sein braucht, wie dies bei den im Zusammenhang mit Tru-cut-Nadeln verwendeten Hohlnadeln der Fall ist; die Stirnseite der Hohlnadel wird vielmehr in einer senkrechten Ebene zu ihrer Achse liegen. Es kann vorgesehen sein, daß die Hohlnadel in ihrer Wandung von der Stirnseite her seitliche Einschnitte aufweist, die an zumindestens einer Längskante ebenfalls zu einer scharfen Kante zugeschliffen sind. Hierdurch ist ein schraubenförmiges Vorbewegen der Hohlnadel und der Federkraft und ein schraubenförmiges Ausschneiden möglich. Bei einer solchen Ausgestaltung wird die Hohlnadel, gegebenenfalls ihr Griffteil oder ein dieses haltenden Halter entlang einer vorrichtungsfesten Schraubennut und durch diese schraubenförmig bewegt.

Weitere bevorzugte Ausgestaltungen sehen vor, daß die Nadeln haltende Aufnahmekörper entgegen der auf sie wirkenden Federkräfte gegeneinander spannbar sind und daß ein Spannteil zum Angreifen an den Aufnahmekörpern ausgebildete Spannansätze mit relativem Abstand zueinander aufweist. Gemäß einer anderen Ausgestaltung ist vorgesehen, daß die Aufnhmekörper an ihren einander abgewandten Querwänden Hinterschneidungen zum Angreifen der Ansätze aufweisen, wobei eine Weiterbildung sich dadurch auszeichnen kann, daß die Aufnahmekörper an ihren einander abgewandten Querwänden Hinterschneidungen zum Angreifen der Ansätze aufweisen.

Zum Festlegen des Stiletts in seiner aktiven Spannstellung kann vorgesehen sein, daß ein von außen betätigbarer Hebel eine Nase zum Halten des Stiletts in aktiver Spannstellung aufweist. Die Festlegung der Hohlnadel in ihrer aktiven Spannstellung erfolgt vorzugsweise dadurch, daß ein die Hohlnadel in aktiver Spannstellung haltender Hebel in axialem Abstand zur Spannstellung des Aufnahmeteils für das Stilett einen Hebelarm aufweist, gegen den ein Ansatz das Aufnahmekörpers für das Stilett unter Federwirkung bringbar ist, um die Hohlnadel aus ihrer aktiven Spannstellung freizugeben.

Insgesamt wird durch die erfindungsgemäße Biopsie-Vorrichtung eine Vorrichtung geschaffen, die durch das gegensätzliche Schießen von Stilett und Hohlnadel, also Zurückziehen des Stiletts und anschließendes Vorschießen der Hohlnadel, gegebenenfalls unter Drehung derselben, jeweils unter Federkraft eine sichere, zügige und schnelle Probenentnahme gewährleistet werden kann, die den Patienten nicht belastet, wobei er insbesondere dazu beiträgt, daß der Durchmesser von Stilett und Hohlnadel sehr gering gewählt werden kann, dennoch aber ein hinreichendes Probenvolumen bzw. eine Probe mit einem hinreichenden Querschnitt entnommen werden kann.

Weitere Vorteile und Merkmale der Erfindung ergeben sich aus den Ansprüchen und aus der nachfolgenden Beschreibung, in der ein bevorzugtes Ausführungsbeispiel der Erfindung unter Bezugnahme auf die Zeichnung im einzelnen erläutert ist. Dabei zeigt bzw. zeigen:
- Figur 1: eine Aufsicht auf die erfindungsgemäße Vorrichtung;
- Figur 2: eine Sicht entsprechend dem Pfeil II in Figur 1 (Figur 2a) bzw. einen Schnitt entsprechend b-b der Figur 2a (Fig.2b) auf bzw. durch den Aufnahmekörper für das Stilett;
- Figur 3: ähnliche Darstellung wie die der Fig.2 des Aufnahmekörpers für die Hohlnadel;
- Figur 4: eine Sicht auf den Haltehebel für die Hohlnadel entsprechend IV der Figur 1.

Die erfindungsgemäße Vorrichtung zur Probenentnahme mittels Biopsie, kurz Biopsievorrichtung 1, weist ein Gehäuse 2 auf, das mittels einer Abdeckung (nicht dargestellt) verschließbar ist.

In dem Gehäuse sind fest zwei Führungsstäbe 3, 4 angeordnet, die sich mit Abstand und parallel zueinander in Längsrichtung des Gehäuses in diesem erstrecken. Auf den Führungsstäben 3, 4 sind gleitbar entlang derselben Aufnahmekörper 11, 31 für ein Stilett 12 bzw. ein Griffteil 13 desselben und eine Hohlnadel 32 bzw. ein Griffteil 33 derselben angeordnet. Das Stilett 12 erstreckt sich durch die Hohlnadel 32 und ragt in völlig eingeschobener Position gerade mit seiner Spitze aus der Hohlnadel 32 heraus. Die Griffteile 13, 33 beider Nadeln 12, 32 werden in Aufnahmeausnehmungen 14, 34 der Aufnahmekörper 11, 31 aufgenommen und gehalten. Die Hohlnadel 32 erstreckt sich (mit dem in ihr aufgenommenen Stilett 12) durch eine Führungsnut 6 in einer vorderen (proximalen) Querwand 7 des Gehäuses 2 aus diesem heraus.

Beide Aufnahmekörper 11, 31 sind in axialer Richtung derart federbelastet, daß sie sich unter freier Federwirkung fortbewegen können und zwar der Aufnahmekörper 11 bis zu einer rückwärtigen (distalen), zur Querwand 7 parallel verlaufenden Querwand 8 des Gehäuses und der Aufnahmekörper 31 bis zu einem in Längs- oder Achsenrichtung verstellbaren und in seiner jeweiligen Position fixierbaren Anschlag 9.

Zur Ausübung der Federwirkung sind jeweils um die Führungsstangen 3, 4 eine Druckfeder 16, 36 angeordnet, die sich jeweils durch eine verbreiterte Ausnehmung 37, 17 des jeweils anderen Aufnahmekörpers 31, 11 hindurcherstrecken und an den einander zugewandten Seiten 18, 38 der Aufnahmekörper anliegen, während sie ihr Widerlager an der vorderen Querwand 7 - für die den Aufnahmekörper 11 beaufschlagende Feder 16 - bzw. an der rückwärtigen Querwand 8 - für die den Aufnahmekörper 31 beaufschlagende Feder 36 - finden.

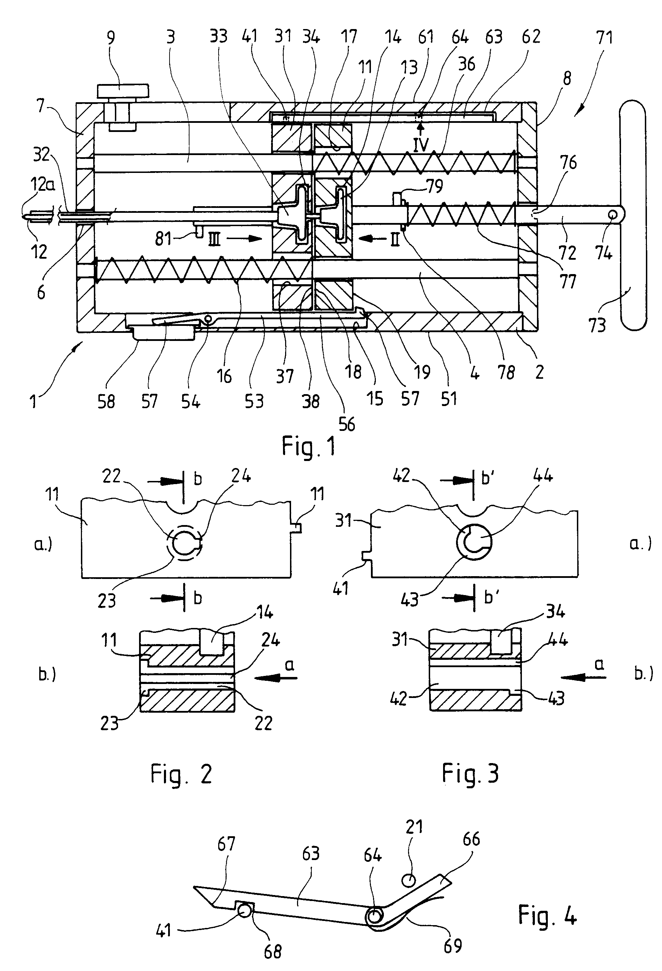

Im dargestellten Ausführungsbeispiel umgibt also die Feder 16 den Führungsstab 4 und findet ihr stationäres Widerlager an der vorderen Querwand 7, ragt mit ihrem anderen Ende durch den verbreiterten Durchbruch 37 des Aufnahmekörpers 13 und rückt mit diesem Ende gegen die dem Aufnahmekörper 31 zugewandte Seite 18 des Aufnahmekörpers 11. Die Feder 36 umgibt den Führungsstab 3, hat ihr stationäres Widerlager an der rückwärtigen Querwand 8, erstreckt sich durch den verbreiterten Durchbruch 17 des Aufnahmekörpers 11 und drückt den Aufnahmekörper 31 durch Anlage an dessen Seite 38 in Richtung auf die vordere Querwand 7. Beide Aufnahmekörper 11, 31 werden entgegen der auf sie wirkenden Federkräfte in der dargestellten Positionen, bei denen ihre einander zugewandten Querwände 18, 38 aneinander anliegen, in der folgenden Weise gehalten:
An einer Längswand 51 des Gehäuses 2 ist (in einer Ausnehmung 52) ein zweiarmiger Hebel 53 um eine Schwenkachse 54 gehalten. Der Hebel 53 weist an einem ersten Hebelarm 56 eine vorragende Nase 57 auf, die die der Querwand 18 des Aufnahmekörpers 11 abgewandte Querwand 19 hintergreift und derart den Aufnahmekörper 11 entgegen der Wirkung der Feder 16 hält. Am dem Hebelarm 56 des Hebels 54 abgewandten Hebelarm 57 greift ein in der Längswand 51 des Gehäuses gelagerter und aus diesem herausragender Betätigungsknopf 58 heraus. Wird dieser Knopf zum Gehäuseinneren hingedrückt, so drückt er auf den Hebelarm 57 und verschwenkt damit den Hebel 53 um sein Schwenklager 54 (in der Sicht der Fig.1) in Uhrzeigersinn, so daß der Hebel 56 mit seiner Nase 57 von dem Aufnahmekörper 11 weggeschwemmt wird und diesen freigibt, so daß er - zusammen mit dem durch ihn gehaltenen Stilett 12 - unter der auf ihn ausgeübten Federwirkung gegen die rückwärtige Querwand 8 des Gehäuses 2 bewegt wird und damit das Stilett 12 mit seiner Spitze 12a in die Hohlnadel 32 zurückgezogen wird.

Der Aufnahmekörper 31 weist an seiner dem Hebel 53 abgewandten Seite einen Ansatz 41 auf. In der Längswandung 61 des Gehäuses 2 ist (in einer Ausnehmung 62) ebenfalls ein doppelarmiger Hebel 63 angeordnet, der über ein Schwenklager 64 verschwenkbar ist (hierzu auch Figur 4). Der doppelarmige Hebel 63 weist an seinem vorderen Hebelarm 57 eine Hinterschneidung in Form einer Nut 68 auf, in die der Ansatz 41 des Aufnahmekörpers 31 eingreift, wodurch der Aufnahmekörper 31 entgegen der auf ihn drückenden Federwirkung in der in Figur 1 dargestellten Spannstellung gehalten wird. Der Hebel 63 wird unter Federwirkung in seine am Ansatz 41 angreifende (in Figur 4 dargestellte) Stellung gedrückt, wobei die Feder 69 am stationären Teil des Schwenklagers 64 angreifen kann und gegen den weiteren Hebelarm 56 des Hebel 53 drückt.

An der dem Hebelarm 63 zugewandten Seite des Aufnahmekörpers 11 ist dieser mit einem dem Ansatz 41 entsprechenden Ansatz 21 versehen. Wenn der Aufnahmekörper 11 in der oben beschriebenen Weise aus seiner Blockierstellung gelöst wird und unter Federwirkung sich gegen die Querwand 8 bewegt, so kommt der Ansatz 21 zunächst zur Anlage am Hebelarm 56, drückt diesen entgegen der Wirkung der Feder 69 (in der Darstellung im Uhrzeigersinn) herunter und hebt damit den Hebelarm 67 von dem Ansatz 41 ab, so daß die Nut 68 den Ansatz 41 und damit den Aufnahmekörper 63 freigibt. Dieser wird sodann unter Federwirkung in Richtung auf die vordere Querwand 7 gedrückt, bis er am Anschlag 9 zum Anliegen kommt. Hierdurch wird mit dem Aufnahmekörper 31 die Hohlnadel 32 in die interessierende Geweberegion weiter vorgeschossen, schneidet damit einen Gewebepfropfen aus dieser, der unter dem durch Zurückziehen des Stiletts 12 in die Hohlnadel bewirkten Unterdruck vom umgebenden Gewebe gelöst und in die Hohlnadel hineingezogen wird.

Zum erneuten Spannen der erfindungsgemäßen Vorrichtung ist ein Spannteil 71 vorgesehen. Dieses Spannteil weist einen Stab 72 sowie einen Griff 73 auf. Der Stab 72 erstreckt sich durch die rückwärtige Querwand 8 in Längsrichtung - parallel zu den Führungsstäben 3, 4 - in das Gehäuse und durch Durchbrüche 22, 42 der Aufnahmekörper 11, 31. Der Griff 73 ist außerhalb des Gehäuses 2 mit dem Stab 72 verbunden und erstreckt sich quer zu diesem. Unmittelbar vor dem Griff 73 ist am Stab 72 ein Ansatz 74 vorgesehen, der in eine entsprechende Ausnehmung 76 der Rückwand 8 eingreifen kann, um so den Griff 73 in einer die Biopsie ermöglichenden Ruhestellung zu halten.

Das Bandteil 71 kann federbelastet sein. In diesem Fall ist um den Stab 72 eine Druckfeder 77 angeordnet, die einerseits ihr stationäres Widerlager an der Querwand 8 findet, andererseits von einem fest mit der Stange 72 verbundenen Widerlager 78 gehalten wird und das Spannteil 71 demgemäß in das Gehäuse 2 hinein und das Griffteil 73 gegen die Querwand 8 drückt.

Mit axialem Abstand zueinander sind am Stab 72 Radialansätze 79, 78 angeordnet. Der Aufnahmekörper 11 weist auf der dem Aufnahmekörper 31 abgewandten Querwand eine Hinterschneidung 23 auf, an der der Ansatz 79 bei Verdrehung der Stange 72 mittels des Griffs 73 um etwa 90° angreifen kann, so daß durch Eindrücken des Griffs 73 und der Stange 72 der Aufnahmekörper 11 von der rückwärtigen Querwand fort auf die vordere Querwand zugedrückt wird, bis er an einer Schräge des Hakens 57 entlang gleitet, diesen dabei zurücktritt und schließlich den Haken 57 hintergreift, so daß er durch diesen in der in der Figur 1 dargestellten aktiven oder Spannstellung gehalten wird. Durch weiteres Verdrehen, beispielsweise wiederum um 90° kann der Ansatz 81 in eine Hinterschneidung 43 am Aufnahmekörper 31 gebracht werden und durch Zurückziehen des Griffs 33 über den Ansatz 81 der Aufnahmekörper 31 wieder in seine aktive oder Spannstellung zurückgezogen werden, bis die Nut 86 am Ansatz 41 des Aufnahmekörpers 31 angreift und diesen damit in der genannten Stellung hält. Die Aufnahmekörper 11, 31 weisen seitlich ihrer Durchbrüche 22, 42 für die Stange 72 Nuten 24 bzw. 44 auf, die erlauben, daß die Aufnahmekörper 11, 31 in der Ruhestellung des Spannteils 71 (bei dem der Ansatz 34 in die Ausnehmung 76 greift) frei über die Ansätze 79, 81 gleiten können und bei ihrer Schießbewegung unter Einwirkung der Federn 16, 36 nicht behindert werden bzw. nicht das Spannteil 71 beaufschlagen.

Der Spannvorgang wird also derart vorgenommen, daß das Spannteil 71 aus dem Gehäuse 2 herausgezogen wird, wobei der Ansatz 79 durch die Nut 24 gleitet, anschließend das Spannteil 71 um 90° verdreht wird, so daß der Ansatz 79 hinter der Hinterschneidung 73 zur Anlage kommt. Das Spannteil 71 wird dann nach innen gedrückt, bis der Vorsprung 57 des Hebels 53 das Aufnahmeteil 11 hintergreift und hält (bei diesem Vorgang ragt der Ansatz 81 nach oben (also in der Darstellung aus der Zeichnungsebene heraus)) und kann daher durch die Nut 44 am Durchbruch 42 hindurchbewegt werden. Ein weiteres Verdrehen um 180° bringt den Ansatz 41 hinter die Hinterschneidung 43 des Aufnahmekörper 31, der durch Zurückziehen ebenfalls in seine durch den Hebel 63 gehaltene Spannstellung gebracht werden kann. Ein weiteres Verdrehen um 90° und Freilassen des Spannteils 71 bringt dieses in der in Figur 1 dargestellten Drehstellung unter der Wirkung der Feder 47 in seine Ruheposition, bei der der Ansatz 74 in die Ausnehmung 76 greift. Anschliessend wird die Biopsie vorgenommen, indem zunächst Hohlnadel 32 und Stilett 12 mit geringfügig aus der Hohlnadel 32 ragender Spitze 12a von Hand durch Vorschieben der gesamten Vorrichtung 1 bis in den interessierenden Biopsiebereich gebracht werden. Daraufhin kann die Biopsie in der schon oben beschriebenen Weise durch Auslösen mittels des Druckknopfs 12 vorgenommen werden und schließlich durch Zurückziehen der gesamten Anordnung der Bioptat aus dem Körper entnommen werden.

## Patentansprüche

1. Vorrichtung zur Probeentnahme mittels Biopsie, mit einer Hohlnadel (32) und einem in dieser geführten Stilett (12), die beide federbeaufschlagt sind, wobei die Hohlnadel (32) unter Federkraft nach vorne schiebbar ist, dadurch gekennzeichnet, daß zur Erzeugung eines Unterdrucks innerhalb der Hohlnadel (32) das Stilett (12) vor dem Vorschießen der Hohlnadel (32) unter Federkraft zurückziehbar ist.

2. Vorrichtung nach Anspruch 1, dadurch gekennzeichnet, daß die Nadeln (12, 32) haltende Aufnahmekörper (11, 31) entgegen der auf sie wirkenden Federkräfte gegeneinander spannbar sind.

3. Vorrichtung nach Anspruch 2, dadurch gekennzeichnet, daß ein Spannteil (71) zum Angreifen an den Aufnahmekörpern (11, 31) ausgebildete Spannansätze (79, 81) mit relativem Abstand zueinander aufweist.

4. Vorrichtung nach Anspruch 3, dadurch gekennzeichnet, daß die Aufnahmekörper (11, 31) an ihren einander abgewandten Querwänden Hinterschneidungen (23, 43) zum Angreifen der Ansätze (79, 81) aufweisen.

5. Vorrichtung nach Anspruch 3 oder 4, dadurch gekennzeichnet, daß die Aufnahmekörper (11, 31) seitlich an beim Durchtreten eines Stabes (72) des Spannteils (71) erlaubenden Bohrungen (22, 42) durch sie hindurchgehende Nuten (24, 44) aufweisen.

6. Vorrichtung nach einem der Ansprüche 1 bis 5, dadurch gekennzeichnet, daß ein von außen betätigbarer Hebel (53) eine Nase (56a) zum Halten des Stiletts (12) in aktiver Spannstellung aufweist.

7. Vorrichtung nach einem der Ansprüche 2 bis 6, dadurch gekennzeichnet, daß ein die Hohlnadel (32) in aktiver Spannstellung haltender Hebel (63) in axialem Abstand zur Spannstellung des Aufnahmeteils (11) für das Stilett (12) einen Hebelarm (66) aufweist, gegen den ein Ansatz (41) des Aufnahmekörpers (11) für das Stilett (12) unter Federwirkung bringbar ist, um die Hohlnadel (32) aus ihrer aktiven Spannstellung freizugeben.

8. Vorrichtung nach einem der vorangehenden Ansprüche, gekennzeichnet durch einen in axialer Längsrichtung verstellbaren Anschlag (9) für das Aufnahmeteil (31) der Hohlnadel (32).

## Claims

1. Device for sampling by means of biopsy, with a hollow needle (32) and a stylet (12) guided therein, both said members being spring-loaded, the hollow needle (32) being forwardly movable under spring tension, characterized in that for producing a vacuum within the hollow needle (32) the stylet (12) is retractable under spring tension prior to the advance of the hollow needle (32).

2. Device according to claim 1, characterized in that the reception members (11, 31) holding the needles (12, 32) can be fixed against one another counter to the spring tensions acting thereon.

3. Device according to claim 2, characterized in that a clamping part (71) has reciprocally relatively spaced clamping attachments (79, 81) for engaging on the reception bodies (11, 31).

4. Device according to claim 3, characterized in that the reception members (11, 31) have on their remote partitions undercuts (23, 43) for the engagement of the attachments (79, 81).

5. Device according to claim 3 or 4, characterized in that the reception members (11, 31) are laterally provided with grooves (24, 44) on bores (22, 42) permitting the passage through the same of a rod (72) of the clamping part (71).

6. Device according to one of the claims 1 to 5, characterized in that a lever (53) operable from the outside has a nose (56a) for holding the stylet (12) in the active clamping position.

7. Device according to one of the claims 2 to 6, characterized in that a lever (63) holding the hollow needle (32) in the active clamping position has a lever arm (66) axially spaced from the clamping position of the reception member (11) for the stylet (12) and against which can be brought under spring action an attachment (41) of the reception member (11) for the stylet (12) in order to release the hollow needle (32) from its active clamping position.

8. Device according to one of the preceding claims, characterized by a stop (9), adjustable in the axial longitudinal direction, for the reception part (31) of the hollow needle (32).

## Revendications

1. Dispositif pour le prélèvement d'un échantillon par biopsie, comprenant une aiguille creuse (32) et un stylet (12) guidé dans celle-ci, montés tous les deux en rappel élastique, l'aiguille creuse (32) pouvant être coulissée vers l'avant sous l'effet d'une force élastique, caractérisé en ce que le stylet (12) est rétracté vers l'arrière par la force élastique avant l'introduction de l'aiguille creuse (32) afin de créer une dépression à l'intérieur de l'aiguille creuse (32).

2. Dispositif selon la revendication 1, caractérisé en ce que des coulisseaux (11,31) renfermant les aiguilles (12,32) sont mis en tension l'un vers l'autre à l'encontre des forces actives élastiques.

3. Dispositif selon la revendication 2, caractérisé en ce qu'une pièce de mise en tension (71) présente pour la préhension des coulisseaux (11,31) des éléments conformés de mise en tension (79,81) disposés à distance relative l'un de l'autre.

4. Dispositif selon la revendication 3, caractérisé en ce que les coulisseaux (11,31) présentent sur leurs faces transversales opposées des évidements (23,43) dans lesquels viennent en prise les saillies (79,81).

5. Dispositif selon la revendication 3 ou 4, caractérisé en ce que les coulisseaux (11,31) présentent latéralement au passage d'une tige (72) du dispositif de mise en tension (71) des perçages ouverts (22,42) traversés par des gorges (24,44).

6. Dispositif selon l'une des revendications 1 à 5, caractérisé en ce qu'un levier (53) actionné de l'extérieur présente un talon (56a) pour maintenir le stylet (12) en position active de tension.

7. Dispositif selon l'une des revendications 2 à 6, caractérisé en ce qu'une bielle (63) maintenant l'aiguille creuse (32) en tension présente, à distance axiale de la position de tension du coulisseau (11) du stylet (12) un bras de levier (66), contre lequel une saillie (41) du coulisseau (11) du stylet (12) peut être amenée élastiquement, pour libérer l'aiguille creuse (32) de sa position de tension active.

8. Dispositif selon l'une quelconque des revendications précédentes, caractérisé par une butée (9) pour le coulisseau (31) de l'aiguille creuse (32) mobile axialement dans le sens de la longueur.
